# EUROPEAN PATENT APPLICATION

(11) **EP 2 927 828 A1**
(43) Date of publication of application: **07.10.2015**
(21) Application number: 15157035.5
(22) Date of filing: 27.02.2015
(51) Int. Cl.: G06F 19/00, G01N 33/50

(54) **APPARATUS AND METHODS FOR MEDICAL TESTING**

(30) Priority: 03.03.2014 JP 2014040284; 28.01.2015 JP 2015014332
(71) Applicant: ARKRAY, Inc., Minami-ku Kyoto-shi Kyoto 601-8045 (JP)
(72) Inventor: Kawabata, Yutaka, Kyoto-shi, Kyoto 6020008 (JP)
(74) Representative: MacDougall, Alan John Shaw

(57) **Abstract**

A measuring apparatus and a measuring method that enable measurement to be performed faster and more accurately in POCT, for example, are provided. The measuring apparatus is provided with a measurement unit for performing measurement processing on a sample, an input unit for inputting data that is associated with the measurement processing by the measurement unit and belongs to one of a plurality of attributes, and a display unit for providing a user with information visually. The display unit displays an individual display pattern having an attribute display portion area for displaying a plurality of attribute display portions that respectively correspond to the plurality of attributes and an input instructing area for visually instructing an operation for inputting the data belonging to the attribute corresponding to one of the plurality of attribute display portions displayed in the attribute display portion area.

## Description

### BACKGROUND OF THE INVENTION

### 1. Field of the Invention:

The present invention relates to a measuring apparatus and a measuring method that perform measurement on samples of biological origin.

### 2. Description of Related Art:

Measurement on samples of biological origin is widely implemented as a means of maintaining the health of living organisms including humans. Point of Care Testing (hereinafter, POCT) is the collective term for testing carried out by healthcare professionals working directly with patients, and is mainly conducted at clinics, hospitals, and the like. It is envisioned that measuring apparatuses employed in POCT will be used by multiple healthcare professionals, and measurement is performed on samples originating from multiple patients. In the case of specialized measuring instruments that persons who perform measurement (hereinafter, "measurers") carry for their own use, the measurer and the patient remain the same. In contrast, in POCT, there are multiple measurers and multiple patients. There is also a relative increase in the types of specimens that are used.

An exemplary measuring apparatus that is used in POCT is disclosed in JP-B2-5227871. A user (measurer) ID, a patient ID, a chip (specimen) ID and the like are input as a plurality of types of data associated with the measurement processing to the measuring apparatus disclosed in JP-B2-5227871. At the time of inputting this data, messages prompting the input of respective data are displayed on a display unit.

### SUMMARY OF THE INVENTION

However, the actual processing for inputting the user (measurer) ID, the patient ID, the chip ID and the like generally involves a different operation for each type of data. There is thus concern that, even when prompted to input data, a measurer with little experience or a measurer who is extremely busy will not input the data properly. Also, since it is assumed that multiple types of data will be handled, a comprehensive awareness of the multiple types of data that are handled while focusing on the task of inputting the individual data is preferable in achieving fast and accurate measurement.

The present invention was conceived under the above circumstances, and an object of the present invention is to provide a measuring apparatus and a measuring method that enable measurement to be performed quickly and with greater accuracy.

A measuring apparatus according to a first aspect of the present invention is provided with a measurement unit for performing measurement processing on a sample, an input unit for inputting data that is associated with the measurement processing by the measurement unit and belongs to one of a plurality of attributes, and a display unit for providing a user with information visually. The display unit displays an individual display pattern having an attribute display portion area for displaying a plurality of attribute display portions that respectively correspond to the plurality of attributes and an input instructing area for visually instructing an operation for inputting the data belonging to the attribute corresponding to one of the plurality of attribute display portions displayed in the attribute display portion area.

In the present invention, "visually instructing" refers to using visually recognizable information forms including characters and sentences, images such as illustrations and photographs, and moving images representing items to be conveyed so that the user understands by viewing these information forms. In the present invention, information forms other than text can be used particularly favorably.

In a preferred embodiment of the present invention, the attribute display portions take a plurality of visually different states, according to an input state of the data belonging to the corresponding attribute.

In a preferred embodiment of the present invention, when input of data belonging to one attribute has been completed in a state where the attribute display portion corresponding to the attribute is displayed in the attribute display portion area, the attribute display portion corresponding to another attribute is displayed in the attribute display portion area, and an operation for inputting data belonging to the other attribute is displayed in the input instructing area.

In a preferred embodiment of the present invention, the display unit displays a collective display pattern having a collective display area for displaying the data belonging to the plurality of attributes that has been inputted.

In a preferred embodiment of the present invention, at least one of the data belonging to the plurality of attributes that is associated with one measurement processing is used as the data that is associated with the next measurement processing.

In a preferred embodiment of the present invention, the data belonging to the plurality of attributes that is associated with one measurement processing is partially or completely initialized prior to the next measurement processing.

In a preferred embodiment of the present invention, the order for inputting the data belonging to the plurality of attributes is at least partially predetermined.

In a preferred embodiment of the present invention, the data belonging to the plurality of attributes is input in an arbitrary order.

In a preferred embodiment of the present invention, an input data display area in which the input data is displayed is further included in the individual display pattern.

In a preferred embodiment of the present invention, when input of data belonging to one attribute has been completed in a state where the attribute display portion corresponding to the attribute is displayed in the attribute display portion area, the data belonging to the attribute input to the input data display area is displayed, in addition to the attribute display portion corresponding to another attribute being displayed in the attribute display portion area, and an operation for inputting data belonging to the other attribute being displayed in the input instructing area.

In a preferred embodiment of the present invention, an operation unit for accepting a user operation that causes a transition from a state where the attribute display portion corresponding to one attribute is displayed in the attribute display portion area and an operation for inputting the data belonging to the attribute is displayed in the input instructing area to a state where the attribute display portion corresponding to another attribute is displayed in the attribute display portion area and an operation for inputting the data belonging to the other attribute is displayed in the input instructing area is provided.

In a preferred embodiment of the present invention, when input of the data belonging to a plurality of specific attributes that are sufficient for execution of the measurement processing, among a plurality of attributes, has been completed, the display unit displays the collective display pattern, and the input data belonging to the plurality of input specific attributes is displayed in the collective display area.

In a preferred embodiment of the present invention, an attribute display portion area for displaying the attribute display portions corresponding to the plurality of specific attributes is further included in the collective display pattern.

In a preferred embodiment of the present invention, confirmation display for confirming whether to transition to display of the collective display pattern is performed on the display unit when input of the data belonging to the plurality of specific attributes has been completed.

In a preferred embodiment of the present invention, the measurement unit includes a specimen insertion portion for accepting a specimen that is provided with the measurement processing, and starts the measurement processing when the specimen is inserted into the specimen insertion portion in a state where the data belonging to the plurality of specific attributes has been inputted.

In a preferred embodiment of the present invention, the measurement unit includes a specimen insertion portion for accepting a specimen that is provided with the measurement processing, and starts the measurement processing when the specimen is inserted into the specimen insertion portion in a state where not all of the data belonging to the plurality of specific attributes has been inputted.

In a preferred embodiment of the present invention, confirmation display for confirming whether to start the measurement processing is performed on the display unit when the specimen is inserted into the specimen insertion portion in a state where the data belonging to the plurality of specific attributes has been inputted.

In a preferred embodiment of the present invention, confirmation display for confirming whether to start the measurement processing is performed on the display unit when the specimen is inserted into the specimen insertion portion in a state where not all of the data belonging to the plurality of specific attributes has been inputted.

In a preferred embodiment of the present invention, the display unit has an interrupt display mode for temporally or spatially interrupting display related to the measurement processing after the measurement processing has started, and performing display for inputting data belonging to the plurality of attributes that has not been inputted.

In a preferred embodiment of the present invention, at least one processing selected from displaying a warning on the display unit, preventing the measurement processing from starting, preventing display of measurement results of the measurement processing, and attaching a warning flag to measurement results of the measurement processing is performed in a case where not all of the data belonging to the specific attributes has been inputted.

In a preferred embodiment of the present invention, the attribute display portion corresponding to specific attributes, among the plurality of specific attributes, whose data has not been inputted, and a measurement attribute display portion corresponding to the measurement processing by the measurement unit is displayed in the attribute display portion area of the collective display pattern, and the measurement attribute display portion takes a plurality of visually different states according to a state of the measurement processing or the measurement result.

In a preferred embodiment of the present invention, the plurality of attributes include at least one selected from a measurer ID identifying a healthcare professional, a patient ID identifying a patient, a specimen ID identifying the specimen, a quality control substance ID identifying a quality control substance that is used in quality control for the measurement processing, a measurement processing ID identifying individual measurement processing, and an attribute relating to a result of the measurement processing.

In a preferred embodiment of the present invention, the measurement processing ID includes the patient ID, and a warning is displayed on the display unit in a case where the patient ID included in the measurement processing ID differs from the input patient ID.

In a preferred embodiment of the present invention, a combination of attributes for which the attribute display portions are displayed in the attribute display portion area, among the plurality of attributes, is settable in advance.

A measuring method according to a second aspect of the present invention is provided with a measurement step of performing measurement processing on a sample, an input step of inputting data that is associated with the measurement processing in the measurement step and belongs to one of a plurality of attributes, and a display step of providing a user with information visually. The display step comprises displaying an individual display pattern having an attribute display portion area for displaying a plurality of attribute display portions that respectively correspond to the plurality of attributes and an input instructing area for visually instructing an operation for inputting the data belonging to the attribute corresponding to one of the plurality of attribute display portions displayed in the attribute display portion area.

In a preferred embodiment of the present invention, in the display step, the attribute display portions take a plurality of visually different states, according to an input state of the data belonging to the corresponding attribute.

In a preferred embodiment of the present invention, in the display step, when input of data belonging to one attribute has been completed in a state where the attribute display portion corresponding to the attribute is displayed in the attribute display portion area, the attribute display portion corresponding to another attribute is displayed in the attribute display portion area, and an operation for inputting data belonging to the other attribute is displayed in the input instructing area.

In a preferred embodiment of the present invention, in the display step, a collective display pattern having a collective display area for displaying the data belonging to the plurality of attributes that has been inputted is displayed.

In a preferred embodiment of the present invention, in the input step, at least one of the data belonging to the plurality of attributes that is associated with one measurement processing is used as the data that is associated with the next measurement processing.

In a preferred embodiment of the present invention, in the input step, the data belonging to the plurality of attributes that is associated with one measurement processing is partially or completely initialized prior to the next measurement processing.

In a preferred embodiment of the present invention, in the input step, the order for inputting the data belonging to the plurality of attributes is at least partially predetermined.

In a preferred embodiment of the present invention, in the input step, the data belonging to the plurality of attributes is input in an arbitrary order.

In a preferred embodiment of the present invention, in the display step, an input data display area in which the input data is displayed is further included in the individual display pattern.

In a preferred embodiment of the present invention, when input of data belonging to one attribute has been completed in a state where the attribute display portion corresponding to the attribute is displayed in the attribute display portion area in the input step, the data belonging to the attribute input to the input data display area is displayed, in addition to the attribute display portion corresponding to another attribute being displayed in the attribute display portion area, and an operation for inputting data belonging to the other attribute being displayed in the input instructing area in the display step.

In a preferred embodiment of the present invention, a user operation that causes a transition from a state where the attribute display portion corresponding to one attribute is displayed in the attribute display portion area and an operation for inputting the data belonging to the attribute is displayed in the input instructing area to a state where the attribute display portion corresponding to another attribute is displayed in the attribute display portion area and an operation for inputting the data belonging to the other attribute is displayed in the input instructing area in the display step is accepted.

In a preferred embodiment of the present invention, when input of the data belonging to a plurality of specific attributes that are sufficient for execution of the measurement processing, among a plurality of attributes, has been completed in the input step, in the display step, the collective display pattern is displayed, and the input data belonging to the plurality of input specific attributes is displayed in the collective display area.

In a preferred embodiment of the present invention, in the display step, an attribute display portion area for displaying the attribute display portions corresponding to the plurality of specific attributes is further included in the collective display pattern.

In a preferred embodiment of the present invention, confirmation display for confirming whether to transition to display of the collective display pattern is performed in the display step when input of the data belonging to the plurality of specific attributes has been completed in the input step.

In a preferred embodiment of the present invention, the measurement step is started when a specimen that is provided with the measurement processing is inserted into a measuring apparatus having a specimen insertion portion for accepting the specimen, in a state where the data belonging to the plurality of specific attributes has been inputted in the input step.

In a preferred embodiment of the present invention, the measurement step is started when a specimen that is provided with the measurement processing is inserted into a measuring apparatus having a specimen insertion portion for accepting the specimen, in a state where not all of the data belonging to the plurality of specific attributes has been inputted in the input step.

In a preferred embodiment of the present invention, in the display step, confirmation display for confirming whether to start the measurement processing is performed when the specimen is inserted into the specimen insertion portion in a state where the data belonging to the plurality of specific attributes has been inputted.

In a preferred embodiment of the present invention, in the display step, confirmation display for confirming whether to start the measurement processing is performed when the specimen is inserted into the specimen insertion portion in a state where not all of the data belonging to the plurality of specific attributes has been inputted.

In a preferred embodiment of the present invention, in the display step, an interrupt display mode is provided for temporally or spatially interrupting display related to the measurement processing after the measurement processing has started, and performing display for inputting data belonging to the plurality of attributes that has not been inputted.

In a preferred embodiment of the present invention, at least one processing selected from displaying a warning in the display step, preventing the measurement processing from starting, preventing display of measurement results of the measurement processing, and attaching a warning flag to measurement results of the measurement processing is performed in a case where not all of the data belonging to the specific attributes has been inputted in the input step.

In a preferred embodiment of the present invention, the attribute display portion corresponding to specific attributes, among the plurality of specific attributes, whose data has not been inputted, and a measurement attribute display portion corresponding to the measurement processing are displayed in the attribute display portion area of the collective display pattern, and the measurement attribute display portion takes a plurality of visually different states according to a state of the measurement processing or the measurement result.

In a preferred embodiment of the present invention, the plurality of attributes include at least one selected from a measurer ID identifying a healthcare professional, a patient ID identifying a patient, a specimen ID identifying the specimen, a quality control substance ID identifying a quality control substance that is used in quality control for the measurement processing, a measurement processing ID identifying individual measurement processing, and an attribute relating to a result of the measurement processing.

In a preferred embodiment of the present invention, the measurement processing ID includes the patient ID, and a warning is displayed on the display unit in a case where the patient ID included in the measurement processing ID differs from the input patient ID.

In a preferred embodiment of the present invention, a combination of attributes for which the attribute display portions are displayed in the attribute display portion area in the display step, among the plurality of attributes, is settable in advance.

According to one mode of the present invention, when inputting data belonging to the plurality of attributes, input can be performed while viewing the display unit on which the individual display pattern is displayed. The attribute display portions of the attribute display portion area allow the types of data that are input items to be grasped at a glance. Also, which of the plurality of attribute display portions the input instructing area is associated with can be recognized. Guidance for inputting data belonging to the selected attribute is provided by the input instructing area. The measurer is thereby able to input data belonging to the individual attributes using input operations suitable for the respective data, while having a comprehensive grasp of the types of input data. Accordingly, the measuring apparatus enables faster and more accurate measurement to be performed.

Other features and advantages of the present invention will become apparent from the following detailed description with reference to the attached drawings.

### BRIEF DESCRIPTION OF THE DRAWINGS

Fig. 1 is a perspective view showing an exemplary measuring apparatus according to the present invention.
Fig. 2 is a perspective view showing the exemplary measuring apparatus according to the present invention.
Fig. 3 is a block diagram showing the exemplary measuring apparatus according to the present invention.
Fig. 4 is a flowchart showing operations of the exemplary measuring apparatus according to the present invention.
Fig. 5 is an illustrative diagram showing a display unit in operations of the exemplary measuring apparatus according to the present invention.
Fig. 6 is an illustrative diagram showing the display unit in operations of the exemplary measuring apparatus according to the present invention.
Figs. 7 are illustrative diagrams showing attribute display portions in operations of the exemplary measuring apparatus according to the present invention.
Fig. 8 is an illustrative diagram showing the display unit in operations of the exemplary measuring apparatus according to the present invention.
Fig. 9 is an illustrative diagram showing the display unit in operations of the exemplary measuring apparatus according to the present invention.
Fig. 10 is an illustrative diagram showing the display unit in operations of the exemplary measuring apparatus according to the present invention.
Fig. 11 is an illustrative diagram showing the display unit in operations of the exemplary measuring apparatus according to the present invention.
Fig. 12 is an illustrative diagram showing the display unit in operations of the exemplary measuring apparatus according to the present invention.
Fig. 13 is an illustrative diagram showing the display unit in operations of the exemplary measuring apparatus according to the present invention.
Fig. 14 is an illustrative diagram showing the display unit in operations of the exemplary measuring apparatus according to the present invention.
Fig. 15 is a flowchart showing operations of the exemplary measuring apparatus according to the present invention.
Fig. 16 is an illustrative diagram showing the display unit in operations of the exemplary measuring apparatus according to the present invention.
Fig. 17 is an illustrative diagram showing the display unit in operations of the exemplary measuring apparatus according to the present invention.
Fig. 18 is an illustrative diagram showing the display unit in operations of the exemplary measuring apparatus according to the present invention.
Fig. 19 is an illustrative diagram showing the display unit in operations of the exemplary measuring apparatus according to the present invention.
Figs. 20 are illustrative diagrams showing the attribute display portions in operations of the exemplary measuring apparatus according to the present invention.
Fig. 21 is an illustrative diagram showing the display unit in operations of the exemplary measuring apparatus according to the present invention.
Fig. 22 is an illustrative diagram showing the display unit in operations of the exemplary measuring apparatus according to the present invention.
Fig. 23 is an illustrative diagram showing the display unit in operations of the exemplary measuring apparatus according to the present invention.

### DESCRIPTION OF THE PREFERRED EMBODIMENTS

Hereinafter, preferred embodiments of the present invention will be specifically described with reference to the drawings.

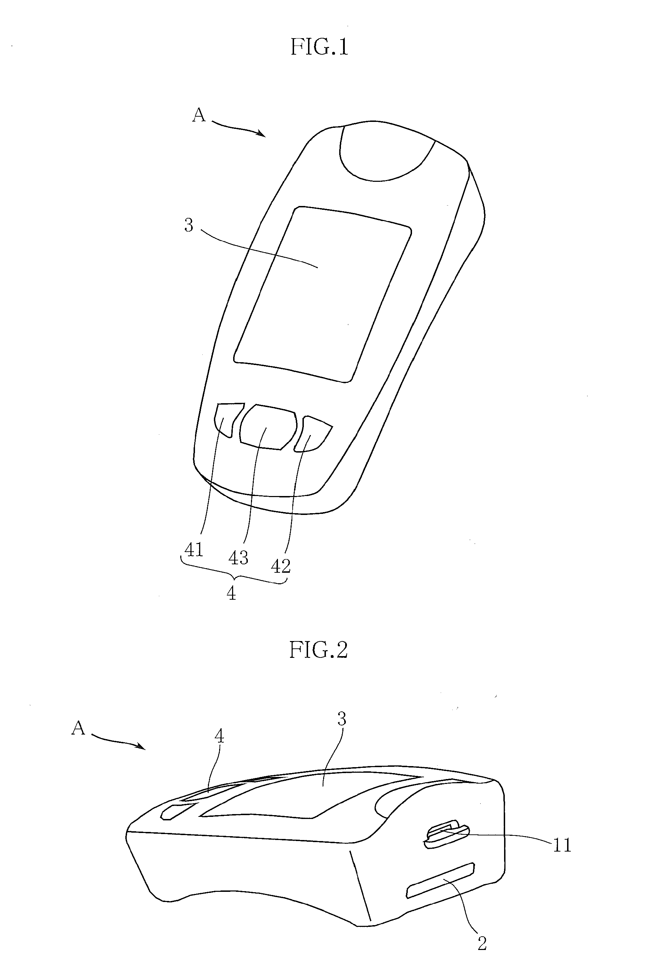

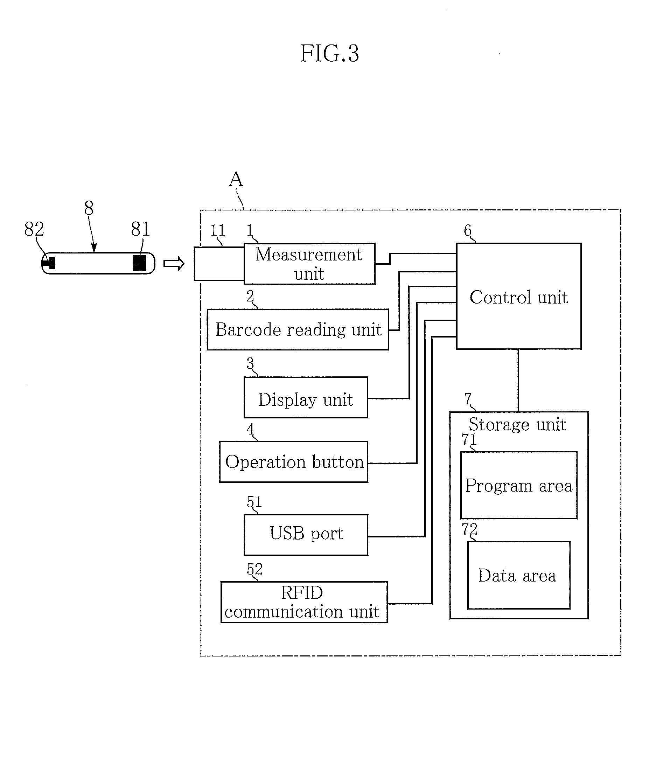

Figs. 1 to 3 show an exemplary measuring apparatus according to the present invention. A measuring apparatus A of the present embodiment is provided with a measurement unit 1, a barcode reading unit 2, a display unit 3, operation button 4, a USB port 51, a wireless communication unit 52, a control unit 6, and a storage unit 7. The measuring apparatus A is used in POCT. In the present embodiment, a configuration in which blood sugar levels are measured as a result of a specimen 8 being loaded in the measuring apparatus A will be described below as an example.

The measuring apparatus A is formed of a size and shape that enables a measurer such as a healthcare professional or the like to perform handheld measurement operations, and is configured such that the measurement unit 1, the barcode reading unit 2, the display unit 3, the operation button 4, the USB port 51, the wireless communication unit 52, the control unit 6, and the storage unit 7 are appropriately attached to or incorporated in a casing made of resin, for example.

The measurement unit 1 performs measurement processing on samples of biological origin. In the present embodiment, the measurement unit 1 performs blood sugar level measurement on blood samples collected from a patient. The measurement unit 1 includes a specimen insertion portion 11. The specimen insertion portion 11 is an opening formed in a portion of the casing, and the specimen 8 is inserted therein. A sensor (not shown) that detects insertion of the specimen 8, for example, is provided at the back of the opening. Note that the specimen 8 includes an electrode 81 and a spotting portion 82, for example. The electrode 81 is a portion that is electrically connected to the measurement unit 1 by contacting a connection terminal (not shown) of the measuring apparatus A. The spotting portion 82 is a portion on which blood is spotted as a sample. Also, a reagent (not shown) is provided on the specimen 8. In the case of performing blood sugar level measurement, the reagent consists of glucose oxidase, which is an enzyme, potassium ferricyanide as an electron acceptor, and the like. With such a configuration, the measurement unit 1 performs blood sugar level measurement processing with a well-known technique on blood that is spotted on the specimen 8 as a sample.

The barcode reading unit 2 corresponds to an exemplary input unit as referred to in the present invention. The measuring apparatus A receives input of data that is associated with the measurement processing and belongs to one of a plurality of attributes. In the present embodiment, examples of the plurality of attributes include a measurer ID identifying a healthcare professional, a patient ID identifying a patient, a specimen ID identifying a specimen, a quality control substance ID identifying a quality control substance that is used in quality control of the measurement processing, and a measurement processing ID identifying individual measurement processing. In the present embodiment, input of this data is performed by the barcode reading unit 2. That is, the data to be input is provided as a barcode printed on media such as cards, wristbands, packages and paper, and the barcode reading unit 2 reads these barcodes. Typical examples of a barcode as referred to here include a one-dimensional barcode constituted by a plurality of striped lines and a two dimensional code constituted by a plurality of dots arrayed horizontally and vertically.

The barcode reading unit 2 is provided with a light-emitting unit, a light-receiving unit and a driver IC, for example. The light-emitting unit emits light for reading, and emits linear beams of light including red light, for example. The light-receiving unit receives reflected light reflected by the medium provided with the barcode, and is constituted by a plurality of light receiving elements arranged in a row or in a matrix. The driver IC forms image data for recognizing a barcode based on an output signal from the light-receiving unit. Alternatively, the driver IC may be configured to output data converted to a barcode as digital numeric data, by performing image analysis of the image data. Also, this numeric conversion function may be provided by the control unit 6.

Note that the input unit as referred to in the present invention is not limited to the barcode reading unit 2, and may be configured to have a plurality of keys known as a numeric keypad that are provided in a mobile phone, for example. Also, the input unit may additionally be provided with a configuration capable of reading barcodes and a configuration having a plurality of keys or a touch panel that allows for flicking.

The display unit 3 provides information to the user (measurer) visually, and a liquid crystal display, an organic electroluminescence display or the like that have a plurality of pixels arranged in a matrix can be favorably used. In the present embodiment, the display unit 3 displays an individual display pattern and a collective display pattern which will be described later. Note that these modes are achieved as a result of the control unit 6 providing a display instruction to the display unit 3 and the display unit 3 displaying predetermined designs, characters and the like based on this display instruction, and are not limited to only the designs, characters and the like that are individually achieved by the display unit 3.

The operation button 4 is a part provided for the user (measurer) to perform decision-making with respect to operations of the measuring apparatus A. In the present embodiment, the operation button 4 is constituted by a direction button 41, a direction button 42, and an OK button 43, for example. The direction button 41 and the direction button 42 are respectively for performing operations for indicating left and right on the display unit 3. The OK button 43 is for performing an operation for permitting an operation or processing indicated by the display of the display unit 3. Also, this configuration can be substituted for a touch panel that allows for flicking.

The USB port 51 and the wireless communication unit 52 are parts that provide an interface between the measuring apparatus A and other devices. Other devices include large multifunctional integrated measurement stations, PCs, and the like that are installed in hospitals, for example. The USB port 51 is a port for realizing wired data communication based on USB standards. The wireless communication unit 52 performs wireless data communication. Although the type of wireless technology is not particularly limited, the case where RFID for performing short-range wireless data communication using electromagnetic waves is employed will be described as an example in the present embodiment. In the case of such an example, the wireless communication unit 52 is equipped with a coil antenna, a driver IC and the like, for example. The wireless communication unit 52 may be configured to perform exchange of information related to measurement, measurement results and IDs selectively or collectively, or in real time.

The control unit 6 is for controlling the operations of the measuring apparatus A, and typically consists of a CPU. The control unit 6 controls the operations of the measurement unit 1, the barcode reading unit 2, the display unit 3, the USB port 51 and the wireless communication unit 52 based on a program stored in the storage unit 7. Also, the control unit 6 receives input signals from the operation button 4. Note that although the following description will be given in the present embodiment assuming that the control unit 6 controls the main operations of the measuring apparatus A, the present invention is not limited thereto. A configuration may be adopted in which the functions performed by the control unit 6 in the present embodiment may be executed by another CPU or the like provided in units such as the measurement unit 1, the barcode reading unit 2 and the display unit 3.

The storage unit 7 includes a program area 71 and a data area 72, and a volatile memory, a nonvolatile memory or the like consisting of a silicon memory, for example, is employed as appropriate. The program area 71 is an area for storing programs that realizes the operations of the measuring apparatus A. The data area 72 is an area for storing data that is associated with measurement. For example, images and the like constituting guidance that is displayed on the display unit 3 in the operations of the measuring apparatus A which will be discussed later may be stored in the data area 72.

Next, operations of the measuring apparatus A will be described below.

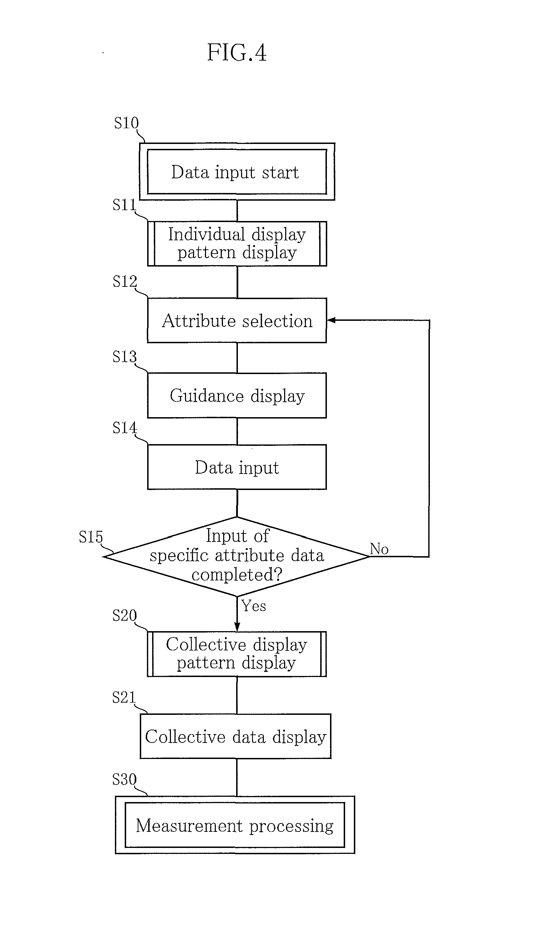

Fig. 4 is a flowchart showing exemplary operations of the measuring apparatus A. The operations shown in this flowchart include input processing for inputting data that is associated with measurement processing (steps S10 to S21) and measurement processing (step S30), and are typical examples of the operations of the measuring apparatus A. Apart from the processing described below, the measuring apparatus A is configured to be able to execute setting processing for setting various condition items relating to operations, browse processing for browsing a plurality of measurement data obtained by measurement processing and the like as appropriate.

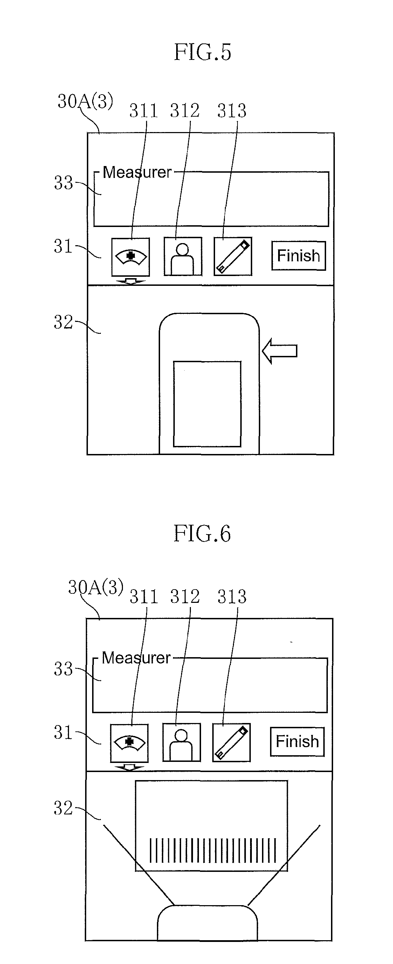

For example, the measuring apparatus A, having ended required initial settings and the like through setting processing, enters a state for executing the input processing according to an instruction from the control unit 6 when powered on (step S10). According to an instruction from the control unit 6, the display unit 3 displays an individual display pattern (step S11). Fig. 5 shows the display unit 3 displaying an individual display pattern 30A. The individual display pattern 30A shown in Fig. 5 has an attribute display portion area 31, an input instructing area 32, and an input data display area 33.

A plurality of attribute display portions 311, 312 and 313 that respectively corresponded to the plurality of attributes are displayed in the attribute display portion area 31. The attribute display portion 311 corresponds to the measurer ID. The attribute display portion 312 corresponds to the patient ID. The attribute display portion 313 corresponds to the specimen ID. In the present embodiment, the attribute display portions 311, 312 and 313 are provided in the form of so-called icons using a design or the like, such that the attribute corresponding to each attribute display portion is understood intuitively. The attribute display portion area 31 of the present embodiment is a band-like area extending horizontally on the display unit 3, and is arranged vertically towards the middle of the individual display pattern 30A. Note that the attribute display portions in the present invention can be portions that display a plurality of attributes in an individually recognizable manner, and, apart from the above-mentioned icons, may be so-called tags that extend partially from the input instructing area or buttons consisting of comparatively simple designs, for example.

Here, the measurer ID, the patient ID and the specimen ID, which are the attributes corresponding to the illustrated attribute display portions 311, 312 and 313, are regarded as a plurality of specific attributes that are sufficient for execution of the measurement processing. That is, the conditions for performing normal measurement processing are satisfied when all the data belonging to the plurality of specific attributes has been inputted and stored in the data area 72 of the storage unit 7. Note that the combination of attribute display portions that are displayed in the attribute display portion area 31 is preset in the above-mentioned setting processing.

Also, in step S12 of Fig. 4, attribute selection for inputting data of the attribute corresponding to one of the plurality of attribute display portions 311, 312 and 313 is performed. A plurality of techniques are provided for performing this attribute selection. First, in the case of a technique in which attribute data is input in a predetermined order, input of data belonging to the attributes corresponding to the respective attribute display portions is input in order of the attribute display portion 311, the attribute display portion 312 and the attribute display portion 313, for example. Next, in the case of a technique in which the input order of the attribute data is not predetermined, the input of data is performed by sequentially selecting the attribute display portions 311, 312 and 313 on the display unit 3 with the operation button 4, for example. More specifically, one of the attribute display portions 311, 312 and 313 is highlighted using the direction button 41 and the direction button 42 to a state for inputting data belonging to the corresponding attribute is input in response thereto or by pressing the OK button 43.

The input instructing area 32 is an area that visually instructs the operation for inputting the data belonging to the attribute corresponding to one of the plurality of attribute display portions 311, 312 and 313 displayed in the attribute display portion area 31. In the present embodiment, the input instructing area 32 is a comparatively large rectangular area, and is arranged towards the lower edge of the display unit 3. The input data display area 33 is an area in which the input data is displayed. In the present embodiment, the input data display area 33 is an elongated rectangular area, and is arranged towards to upper edge of the individual display pattern 30A. The input instructing area 32 can visually instruct the operation for inputting the data belonging to the attribute, and a configuration may be adopted in which the input instructing area 32 appears in a pop-up manner by selecting an attribute display portion, and visually presents the operation for inputting the data belonging to the corresponding attribute.

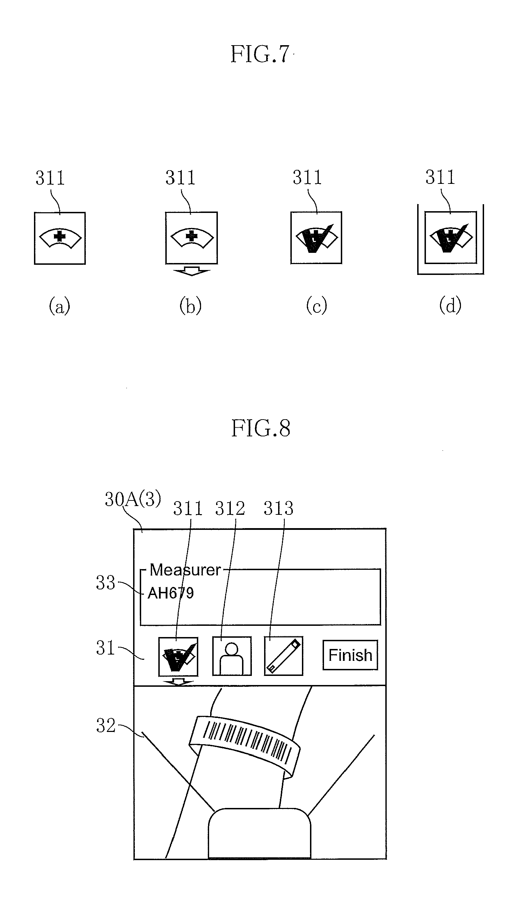

Also, the attribute display portions 311, 312 and 313 are configured to take a plurality of visually different states, according to the input state of the data belonging to the corresponding attribute. Figs. 7 are diagrams illustrating a plurality of visually different states giving the attribute display portion 311 as an example. The attribute display portion 311 shown in Fig. 7(a) is shown to be a state where the measurer ID, which is the corresponding attribute, has not been inputted. Also, this attribute display portion 311 is represented by only a form enclosed in a rectangular shape. This indicates that neither the input instructing area 32 nor the input data display area 33 are performing display relating to the measurer ID. The attribute display portion 311 shown in Fig. 7(b) is shown to be a state where input of the measurer ID can be accepted. Also, this attribute display portion 311 includes a form enclosed in a rectangular shape and a downward arrow, and is configured to be connected to the area below. This indicates that this attribute display portion 311 is associated with the input instructing area 32 that is located below the attribute display portion area 31, and, more specifically, that the operation for inputting the measurer ID corresponding to the attribute display portion 311 is being visually instructed in the input instructing area 32. The attribute display portion 311 shown in Fig. 7(c) is shown to be in a state where input of the measurer ID corresponding to the attribute display portion 311 has been completed. For example, in the case of the measuring apparatus A provided with a display unit 3 capable of color display, the attribute display portions 311 shown in the attribute display portion 311 of Figs. 7(a) and (c) can be clearly distinguished from each other by differentiating the respective colors. In the present embodiment, a symbol evoking an image of completion is given to the attribute display portion 311 shown in Fig. 7(c), in consideration of gray scale representation. The attribute display portion 311 shown in Fig. 7(d) is shown to be in a state where input of the corresponding measurer ID has been completed. Also, this attribute display portion 311 is configured such that a peripheral portion is connected to an information area, in addition to having a form enclosed in a rectangular shape. This indicates that the attribute display portion 311 is associated with the input data display area 33 that is located above the attribute display portion area 31, and, more specifically, indicates that the result of inputting the measurement ID is displayed in the input data display area 33. The plurality of states shown in Figs. 7(a) to (d) are similarly employed for the attribute display portion 312, the attribute display portion 313, and other attribute display portions of the same type.

On the display unit 3 shown in Fig. 5, a state where the attribute display portion 311 corresponding to the measurer ID was selected in the above step S12 is shown. The control unit 6 executes step S13 of Fig. 4. In this step, the attribute display portion 311 takes the state of Fig. 7(b), and the association with the input instructing area 32 is indicated. An image that shows the measuring apparatus A being held and prompts a predetermined operation is displayed in the input instructing area 32. Upon the measurer performing this operation in accordance with this guidance, the control unit 6 recognizes completion of the operation, and displays guidance for the next operation in the input instructing area 32 of the display unit 3. Fig. 6 prompts the measurer to irradiate a card provided with a barcode that includes the measurer ID with red light emitted from the barcode reading unit 2. Upon the measurer performing an operation in accordance with this guidance, the control unit 6 causes the barcode reading unit 2 to perform processing for reading the barcode. This is the data input processing of step S14 in Fig. 4. The control unit 6 stores the digital numeric data sent from the barcode reading unit 2 in the data area 72 of the storage unit 7 as the measurer ID. Also, the control unit 6 changes the state of the attribute display portion 311 to the state shown in Fig. 7(d), as shown in Fig. 8. At the same time, the control unit 6 displays the input measurer ID in the input data display area 33. Data input processing (step S14) regarding the measurer ID, which is one attribute, is thereby completed.

Next, the control unit 6 executes step S15 of Fig. 4. In this step, the control unit 6 judges whether input of all of the specific attributes, namely, the measurer ID, the patient ID and the specimen ID, has been completed. In the case where there is a specific attribute that has not been inputted, the control unit 6 executes steps S12, S13 and S14 with regard to this other attribute (step S15: No). In the attribute selection in step S12, the control unit 6 may perform the selection automatically in a predetermined order, or the measurer may make an arbitrary selection through operation of the operation button 4 or the like.

Fig. 8 shows the individual display pattern 30A in a state where the patient ID was selected as a result of such attribute selection (step S12). As described above, the result of inputting the measurer ID is displayed in the input data display area 33, and the state of the attribute display portion 311 changes accordingly. The attribute display portion 312 changes to a state of accepting input, and guidance for inputting the patient ID is displayed in the input instructing area 32. In the example shown in Fig. 8, the guidance prompts the measurer to irradiate a wristband provided with a barcode that includes the patient ID with red light emitted from the barcode reading unit 2. Upon the measurer performing an operation in accordance with this guidance, the control unit 6 causes the barcode reading unit 2 to perform processing for reading the barcode (step S14), and stores the measurer ID in the data area 72 of the storage unit 7.

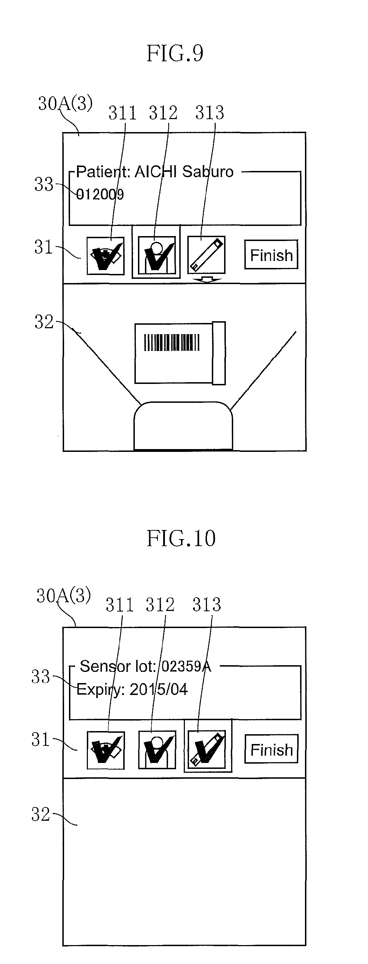

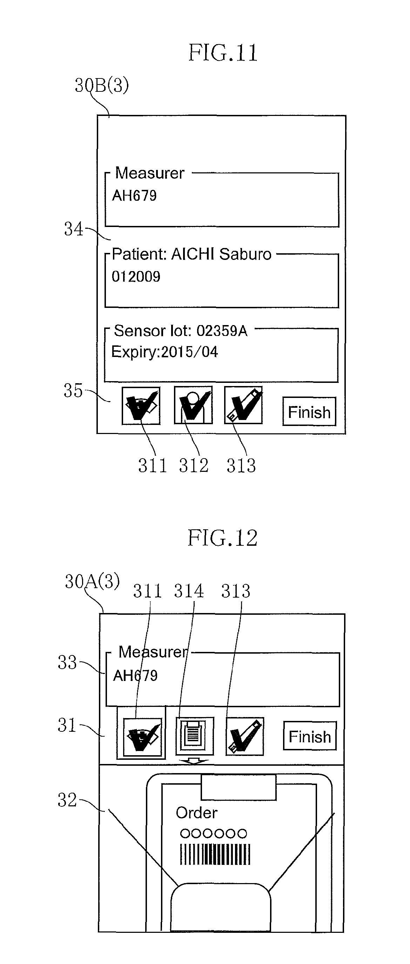

Fig. 9 shows the individual display pattern 30A in a state where the input patient ID is displayed in the input data display area 33 and the attribute display portion 312 has changed accordingly. The name of the patient corresponding to the input patient ID is displayed in the input data display area 33. For example, a data table that includes information such as a plurality of alphanumeric characters that are included in the patient ID and patient names corresponding to this information is pre-stored in the data area 72 of the storage unit 7. The control unit 6 refers to the data table with regard to the input patient ID, and displays the corresponding patient name on the display unit 3. Note that the patient ID may also include the patient name together with a plurality of alphanumeric character information. Also, Fig. 9 shows a state where the above-mentioned steps S15 and S12 have been executed, and the specimen ID has been selected. The attribute display portion 313 has changed to a state of receiving input, and guidance for inputting the specimen ID is displayed in the input instructing area 32. In the example shown in Fig. 9, the guidance prompts the measurer to irradiate a package provided with a barcode that includes the specimen ID with red light emitted from the barcode reading unit 2. Upon the measurer performing an operation in accordance with this guidance, the control unit 6 causes the barcode reading unit 2 to perform processing for reading the barcode (step S14), and stores the specimen ID in the data area 72 of the storage unit 7. Also, in addition to the specimen ID, the barcode provided on the package may also include the expiration date of the packaged specimen, a correction code that is provided with correction of measurement results and the like as appropriate. Fig. 10 takes a state where the input specimen ID is displayed in the input data display area 33, and the attribute display portion 313 has changed accordingly. Note that, in the state shown in Fig. 9, the patient ID that had already been inputted is displayed in the input data display area 33, and guidance for inputting the specimen ID, which is a separate ID different from the patient ID, is displayed in the input instructing area 32. Thus, in the measuring apparatus A of the present embodiment, the input result relating to a certain attribute and guidance relating to another attribute can display at the same time on a single screen, and, as aforementioned, the input state of the data belonging to the attributes can also be displayed on the same screen. Other examples of such a display mode include a display mode shown in Fig. 12 which will be discussed later, for example.

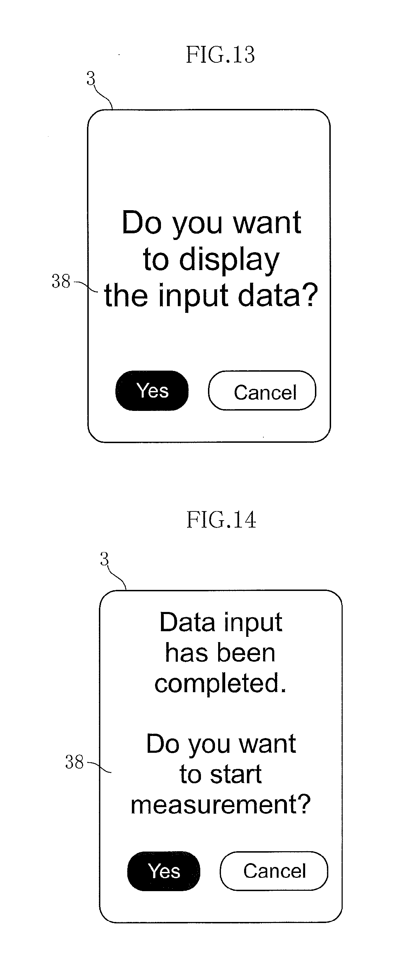

As shown in Fig. 4, in the case where input of all the specific attributes has been completed (step S15: Yes), the control unit 6 advances to step S20. Execution of step S20 may be started automatically by the control unit 6, or a confirmation display area 38 shown in Fig. 13, for example, may be displayed on the display unit 3, and the measurer may be prompted to permit the processing to advance to step S20 with the OK button 43 of the operation button 4.

Fig. 11 shows a state where the control unit 6 displays a collective display pattern 30B on the display unit 3. The collective display pattern 30B has a collective display area 34 and an attribute display portion area 35. The collective display area 34 is an area in which a plurality of specific attributes, namely, the measurer ID, the patient ID and the specimen ID, that have been inputted are collectively displayed. In the present embodiment, the measurer ID, the patient ID and the specimen ID are arrange so as to be aligned in the vertical direction (step S21). The attribute display portion area 35 is an area in which the attribute display portions 311, 312 and 313 corresponding to the plurality of input specific attributes, namely, the measurer ID, the patient ID and the specimen ID, are displayed. Given that the measurer ID, the patient ID and the specimen ID have been inputted, and the collective display pattern 30B is not provided with the input instructing area 32 or the input data display area 33 that are associated with individual attributes, these attribute display portions 311, 312 and 313 take the state shown in Fig. 7(c).

The plurality of specific attributes can be variously set depending on the contents of the measurement processing, the environment, such as a hospital, in which the measuring apparatus A is used or an operation for which the measuring apparatus A is used, and the type and number of specific attributes is determined arbitrarily. Fig. 12 shows an example of the individual display pattern 30A in the case where a measurer ID, an order ID and a specimen ID are set as the plurality of specific attributes. The attribute display portions 311, 313 and 314 are displayed in the attribute display portion area 31 of the individual display pattern 30A. The attribute display portion 314 corresponds to the order ID. The order ID is an ID for identifying individual measurement processing. The order ID may also include numbers signifying date-time, sequence or the like, and may also further include an ID showing the patient from whom the sample to be measured in the measurement processing originates.

As shown in Fig. 4, when step S21 ends, the control unit 6 executes measurement processing (step S30). Execution of step S30 may be started automatically by the control unit 6, or a confirmation display area 38 shown in Fig. 14, for example, may be displayed on the display unit 3, and the measurer may be prompted to permit the processing to advance to step S30 with the OK button 43 of the operation button 4.

In the measurement processing of step S30, the control unit 6 may appropriately display an image showing the operation procedure for executing measurement processing on the display unit 3 as guidance. Operations for executing the measurement processing include inserting an analysis tool 8 and spotting blood on the spotting portion 82 of the analysis tool 8, for example. Also, since the processing for computing the blood sugar level in the measurement unit 1 requires a predetermined amount of time, the control unit 6 may display a waiting time image, for example, on the display unit 3. Also, after the blood sugar level is obtained, the control unit 6 may display the measurement result on the display unit 3. Also, in addition to representing the obtained blood sugar level numerically, display that reports to the measurer that the blood sugar level was a comparatively high value or that the blood sugar level was a comparatively low value may be performed.

As described above, when the sample of a certain patient has been measured, a blood sugar level as a single measurement result is obtained in the measuring apparatus A. The control unit 6 stores this blood sugar level in the data area 72 of the storage unit 7 together with the measurer ID, the patient ID, the specimen ID, the order ID, and the like. As a result of repeating the above processing for a plurality of samples, a plurality of measurement results are stored in the data area 72 of the storage unit 7 of the measuring apparatus A. After measuring a certain number of samples, the measurer transfers these measurement results to a large multifunctional integrated measurement station, a PC or the like installed in a hospital, for example, using the USB port 51 or the wireless communication unit 52. In this integrated measurement station or PC, a large number of measurement results are managed in accordance with operational rules, and are utilized to maintain the health of patients.

Note that when ending one measurement processing and performing the next measurement processing, the data belonging to the plurality of specific attributes may all be maintained, may be partially initialized, or may all be initialized. Also, it is not necessary to initialize the data. Such initialization of part or all of the data can be performed automatically or manually according to a set procedure. For example, in the case where one measurer performs measurement processing on the samples of a plurality of patients, the measurer ID is maintained. On the other hand, in the case where one series of measurements has ended and the next measurer is unknown, all the data belonging to the plurality of specific attributes is initialized. Such a selection can desirably be made as appropriate through settings performed by the measuring apparatus A.

Next, the workings of the measuring apparatus A will be described.

According to the present embodiment, when inputting data belonging to a plurality of attributes, as illustrated in Fig. 5, input can be performed while viewing the display unit 3 on which the individual display pattern 30A is displayed. The attribute display portions 311, 312 and 313 of the attribute display portion area 31 allow the types of data that are input items to be grasped at a glance. Also, which of the attribute display portions 311, 312 and 313 the input instructing area 32 is associated with can be recognized. Guidance for inputting data belonging to the selected attribute is provided by the input instructing area 32. The measurer is thereby able to input data belonging to the individual attributes using input operations suitable for the respective data, while having a comprehensive grasp of the types of input data. Accordingly, the measuring apparatus A enables faster and more accurate measurement to be performed.

As shown in Figs. 7, the attribute display portions 311, 312 and 313 take a plurality of visually different states according to the input state of data belonging to the corresponding attribute. The user is thus able to grasp the state of the data of the selected attribute at a glance, that is, whether the data has not been inputted, is waiting to be input, or has been inputted. Also, states in which the attribute display portions 311, 312 and 313 are associated with the input instructing area 32 and the input data display area 33 that are adjacent to the attribute display portion area 31 are shown visually. Even in the case of a measurer who is not experienced in operating the measuring apparatus A, he or she will thereby be able to intuitively understand, with regard to data belonging to a certain attribute, that guidance is displayed in the input instructing area 32 or that the input result is displayed in the input data display area 33. Also, the combination of the attribute display portions 311, 312 and 313 to be displayed in the attribute display portion area 31 can be preset. It is thereby possible to appropriately select data to be input, even when the user is not experienced in operating the measuring apparatus A. Also, there is an advantage in that data input can be executed smoothly and exhaustively, by making the user aware of the type of combination.

As shown in Figs. 7 and 8, when input of the measurer ID corresponding to the attribute display portion 311 has been completed, the result of inputting the measurer ID is displayed in the input data display area 33. Also, the state of the attribute display portion 312 corresponding to the patient ID, which is the next selection attribute, changes, and guidance relating to input of the patient ID is displayed in the input instructing area 32. The task of inputting data belonging to the plurality of attributes can thereby be continued smoothly. Also, in cases such as where the input data happens to be erroneous, the measurer will immediately be made aware of this fact.

By being able to select the attribute display portions 311, 312 and 313 in an arbitrary order, the measurer is able to perform the input operation in an order that suits him or her. As a result of the attribute display portions 311, 312 and 313 being displayed in the attribute display portion area 31, an arbitrary attribute display portion can be easily selected, and the attribute display portion that is selected can be recognized immediately.

On the other hand, by adopting a configuration in which the attribute display portions 311, 312 and 313 are selected in a predetermined order, unintentionally failing to input data belonging to one of the attributes can be prevented. Accordingly, all of the data can be appropriately input without omission when performing measurement of a large number of samples, for example.

In a configuration that automatically transitions from inputting one attribute to inputting another attribute, the series of input operations can be performed smoothly. On the other hand, in a configuration that requests the measurer to give permission using the operation button 4 in transitioning from inputting one attribute to inputting another attribute, it is possible to avoid unintentionally transitioning to another attribute.

As shown in Fig. 11, when input to all of the attribute display portions 311, 312 and 313 has been completed, the display unit 3 displays the collective display pattern 30B. The measurer can thereby check the respective input data by referring to the collective display area 34. Also, the measurer is able to intuitively grasp that data belonging to all the specific attributes has been completed by referring to the attribute display portion area 35.

A configuration may be adopted in which transition from display of the individual display pattern 30A to display of the collective display pattern 30B is performed automatically or in which the measurer is requested to give permission using the operation button 4. For example, even if input to all of the attribute display portions 311, 312 and 313 has not been completed, the measurer is, for example, able to transition to display of the collective display pattern 30B using the operation button 4.

In the case where permission is given to maintain all of the data belonging to the plurality of specific attributes when one measurement processing ends and the next measurement processing is to be performed, maintaining this data enables the time required for data input to be shortened. On the other hand, by initializing some or all of the data belonging to the plurality of specific attributes, use of erroneous data can be prevented.

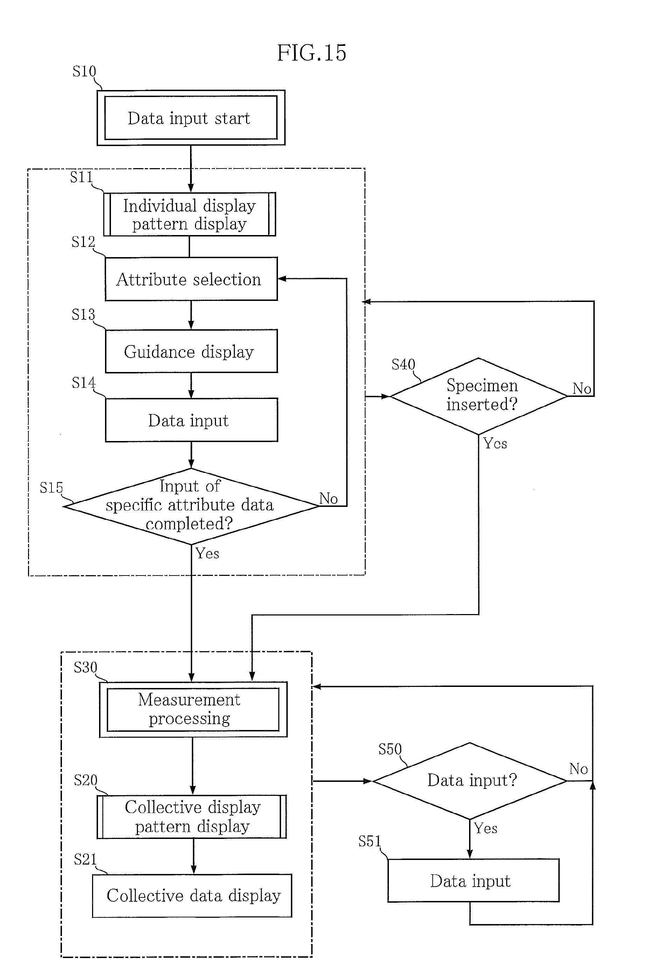

Fig. 15 is a flowchart showing another example of the operations of the measuring apparatus A. In this example, data input processing (steps S11 to S15) using the individual display pattern 30A can be executed in a similar manner. In this example, processing for transitioning to the measurement processing (step S30) differs from the above-mentioned example. Also, a difference with the above-mentioned example is that data input and data display processing can be executed after measurement processing (step S30) has started.

In this example, step S40 for detecting insertion of the specimen 8 is performed as an interrupt during execution of steps S11 to S15, either continuously or for a predetermined time interval. When the specimen 8 is inserted into the specimen insertion portion 11 during execution of steps S11 to S15, that is, in a state where not all of the plurality of specific attributes have been inputted (step S40: Yes), the control unit 6 transitions to the measurement processing (step S30). In the case where insertion of the specimen 8 is not detected (step S40: No), steps S11 to S15 are executed.

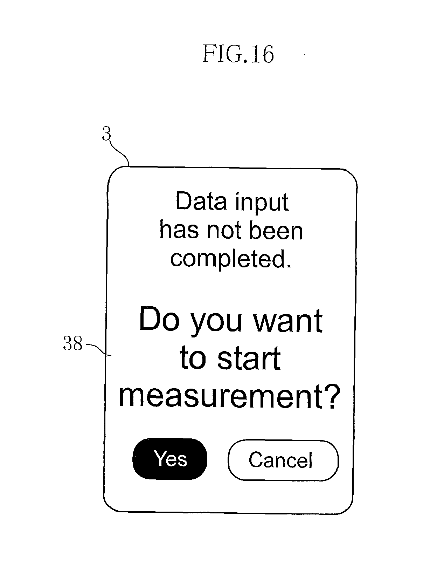

When the specimen 8 is inserted into the specimen insertion portion 11 (step S40: Yes) and the processing transitions to the measurement processing (step S30), the transition may be performed automatically in response to an instruction from the control unit 6 or the measurer may be requested for permission. Fig. 16 shows an example of the display unit 3 in the case of requesting the measurer's permission. In this state, the specimen 8 is inserted in a state where not all of the plurality of specific attributes have been inputted. In this case, the confirmation display area 38 that confirms with the measurer that data input has not been completed and that measurement processing will be started in this state is displayed on the display unit 3. Also, the interrupt of step 40: Yes may be executed as a result of the specimen 8 being inserted into the specimen insertion portion 11 at a point in time at which input of all of the plurality of specific attributes has been completed.

Measurement processing that is started in a state where not all of the plurality of specific attributes have been inputted is defined as urgent blood sugar measurement. Guidance for executing measurement processing, waiting time display and result display may also be displayed on the display unit 3 as appropriate in this urgent blood sugar measurement.

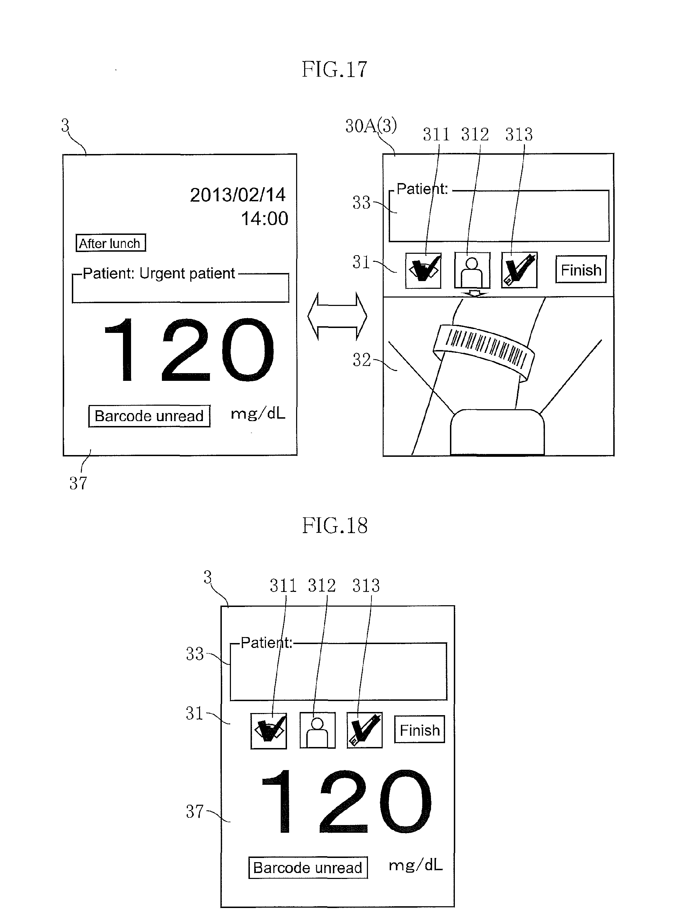

In this example, as shown in Fig. 15, a configuration is adopted in which, after the measurement processing (step S30) has started, data input processing (steps S50 and S51) can be executed as an interrupt. It may be judged in step S50 that data input (step S51) is to be performed (step S50: Yes), using a technique in which the control unit 6 detects whether a predetermined operation has been performed using the operation button 4 after the measurement processing (step S30) was started, for example. Alternatively, as shown in Fig. 17, an interrupt display mode for temporally interrupting display of the display unit 3 on which the measurement result display area 37 is displayed for measurement processing (step S30) (left side in Fig. 17: example in which measurement result is displayed) and displaying the individual display pattern 30A (right side in Fig. 17) may be employed. The measurer inputs data that has not been inputted by performing a predetermined operation at the timing at which the individual display pattern 30A is displayed, while carrying out the urgent blood sugar measurement.

Also, as this interrupt display mode, as shown in Fig. 18, a configuration may be adopted in which display of the display unit 3 for measurement processing (step S30) is spatially interrupted and display for inputting data that has not been inputted is performed. In the example shown in Fig. 18, the measurement result display area 37 is displayed towards the lower edge of the display unit 3, and the attribute display portion area 31 and the input data display area 33 are displayed towards the upper edge of the display unit 3. In the attribute display portion area 31, the attribute display portion 311 and the attribute display portion 313 have been inputted, while the attribute display portion 312 has not been inputted. Also, the word "patient" corresponding to the attribute display portion 312 is displayed in the input data display area 33, and the patient ID, which is the contents, is blank, indicating that data has not been inputted. In the state shown in Fig. 18, data that has not been inputted is inputted by the measurer performing a predetermined operation.

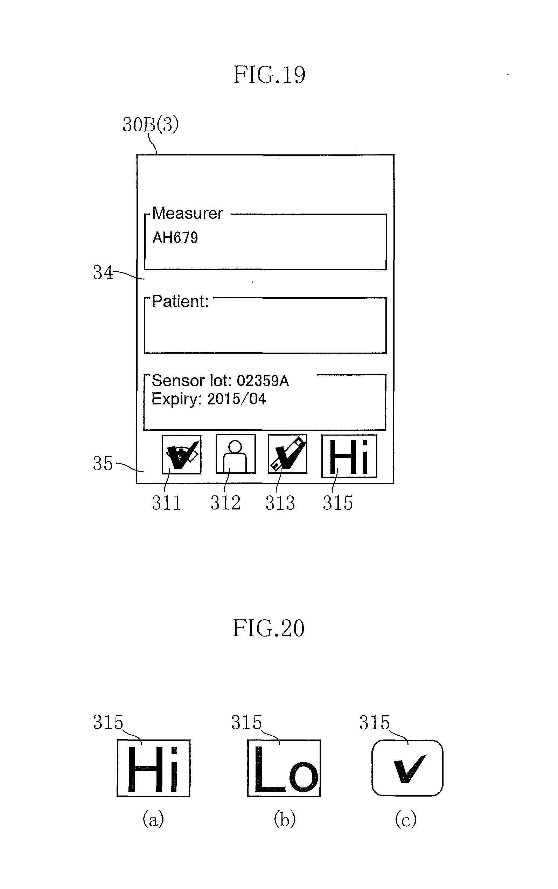

In the measurement processing (step S30) shown in Fig. 15, when data input (step S51) is executed and the plurality of specific attributes have all been inputted, a similar state to the above-mentioned normal measurement processing will be achieved. In this case, result display may be executed, for example. On the other hand, in the case where the measurement processing (step S30) of Fig. 15 is ended in a state where not all of the plurality of specific attributes have been inputted, the control unit 6 may perform collective data display (step S21) in the collective display pattern display (step S20). Fig. 19 shows the display unit 3 in collective data display (step S21). In this case, the display unit 3 is in a state of having the collective display area 34 and the attribute display portion area 35. Data belonging to the plurality of specific attributes is displayed in the collective display area 34. In the example shown in Fig. 19, the measurer ID and the specimen ID have been inputted, and the patient ID has not been inputted. Thus, the data of the measurer ID and the specimen ID is thus displayed in the collective display area 34, and the patient ID is blank. Alternatively, fixed information whose setting can be changed may be displayed for each ID.

Also, the attribute display portions 311, 312 and 313 and the measurement attribute display portion 315 are displayed in the attribute display portion area 35. The outer appearance of the attribute display portion 311 and the attribute display portion 313 corresponds to data having been inputted. The outer appearance of the attribute display portion 312 corresponds to data having not been inputted. The measurement attribute display portion 315 corresponds to the measurement result obtained in the measurement processing. The measurement attribute display portion 315 takes a plurality of visually different states according to a state of the measurement processing or the measurement result. Figs. 20 show exemplary outer appearances of the measurement attribute display portion 315. Fig. 20(a) shows the measurement attribute display portion 315 in the case where the measurement result is comparatively high. Fig. 20(b) shows the measurement attribute display portion 315 in the case where the measurement result is comparatively low. Fig. 20(c) shows the measurement attribute display portion 315 in the case where the measurement result is normal, or alternatively where the measurement processing has simply been completed. The measurement attribute display portion 315 is thus able to express a state of the measurement processing or the measurement result in a quantitative or qualitative manner.

Thus, the display unit 3 shown in Fig. 19 allows the measurer to recognize that the patient ID has not been inputted and that the measurement result is comparatively high. Here, as shown in Fig. 15, the control unit 6 can also perform steps S50 and S51 as an interrupt in steps S20 and S21. Transition from the state shown in Fig. 19 to data input (step S51) can be carried out by performing the temporal interrupt shown in Fig. 17 or a predetermined operation using the operation button 4.

According to such an embodiment, as shown in Fig. 15, even during data input in accordance with the individual display pattern 30A, data input can be intentionally suspended by the measurer by inserting the specimen 8 into the specimen insertion portion 11, and processing can be transitioned to measurement processing. For example, in a clinic or hospital in which POCT is performed, a situation may arise where another sample needs to be measured urgently when measurement of a large number of prepared samples is being conducted. In such a case, measurement can be appropriately implemented as the above-mentioned urgent blood sugar measurement. Also, when the measurer inserts the specimen 8 into the specimen insertion portion 11 at the point in time at which the plurality of specific attributes have all been inputted, processing transitions to the measurement processing. Since inserting the specimen 8 after input has been completed is a natural order of operations for the measurer, the measurement processing can be started more smoothly. Note that, in Fig. 15, processing for storing measurement data in the data area 72 of the storage unit 7 and processing for updating various data follow on from the measurement processing (step S30), the collective display pattern display (step S20), and the collective data display (step S21). Even when performing the above processing, a data input (step S50) interrupt is possible. Thus, a data input (step S50) interrupt is possible even before or after measurement data display and before or after measurement data updating. Also, the measurer can be prompted to confirm whether to start measurement processing even when all of the plurality of specific attributes have not been inputted, by displaying the confirmation display shown in Fig. 16 on the display unit 3.

As shown in Figs. 17 and 18, the interrupt display mode for temporally or spatially displaying an input-related screen as an interrupt is implemented. The measurer can thereby be prompted to input data that has not been inputted (patient ID corresponding to the attribute display portion 312 in the illustrated example), after the start of urgent blood sugar measurement.

As shown in Fig. 19, in the collective display pattern 30B after the urgent blood sugar measurement has ended, the attribute display portion 312 is displayed in the attribute display portion area 35 in a state where data has not been inputted. Also, in the collective display area 34, the patient ID is blank. The measurer can thereby be reliably made aware of which attributes were not input when measurement was performed. Also, the measurement attribute display portion 315 of the attribute display portion area 35 takes a plurality of states corresponding to the measurement result as shown in Figs. 20. The measurer is thereby able to qualitatively grasp the measurement result, while checking the list of data displayed in the collective display area 34 of the display unit 3 in Fig. 19.

As stated above, the measuring apparatus A is able to perform normal blood sugar measurement in a state where all of the plurality of specific attributes have been inputted, and is able to perform urgent blood sugar measurement in a state where not all of the plurality of specific attributes have been inputted. Furthermore, in an situation in which normal blood sugar measurement should be performed, the measuring apparatus A can take the following measures, in order to avoid urgent blood sugar measurement being performed against the measurer's wishes.

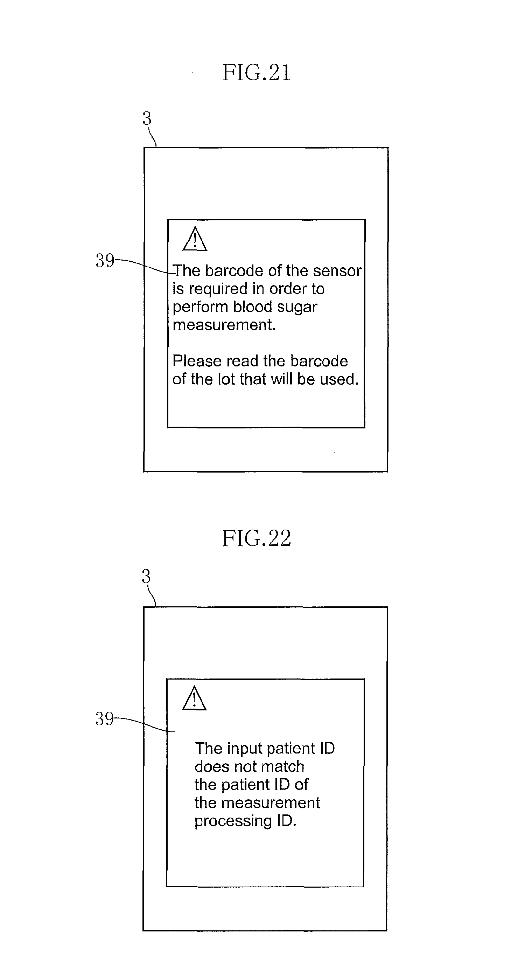

For example, if the measurer tries to perform measurement processing in a state where the specimen ID has not been inputted, the control unit 6 displays a warning display area 39 shown in Fig. 21 on the display unit 3. The measurer is thereby made aware of the fact that there is specific attribute data that has not been inputted, and is prompted to input the data. Alternatively, in the case where measurement processing was performed in a state where all of the plurality of specific attributes were not input, the control unit 6 may prevent display of the measurement data of this measurement processing. Also, the control unit 6 may display a result with a flag indicating that that not all of the data was input attached to this measurement result.

Also, which attributes to employ as the plurality of specific attributes is arbitrary. For example, the plurality of specific attributes may also include the patient ID and the order ID. Furthermore, the order ID in this case may be configured to include the patient ID. According to such a configuration, the measurer is able to designate the patient to be measured with the patient ID that is included in the order ID, and is also able to check whether the patient to undergo the actual measurement is the correct patient. For example, in the case where the patient ID included in the order ID differs from the patient ID read in the wristband by the barcode reading unit 2, the patient that is going to be measured could possibly be the wrong patient. The control unit 6, in such a case, may perform display indicating that the read patient ID does not match the patient ID included in the order ID, as a warning in the warning display area 39 of the display unit 3, as shown in Fig. 22.

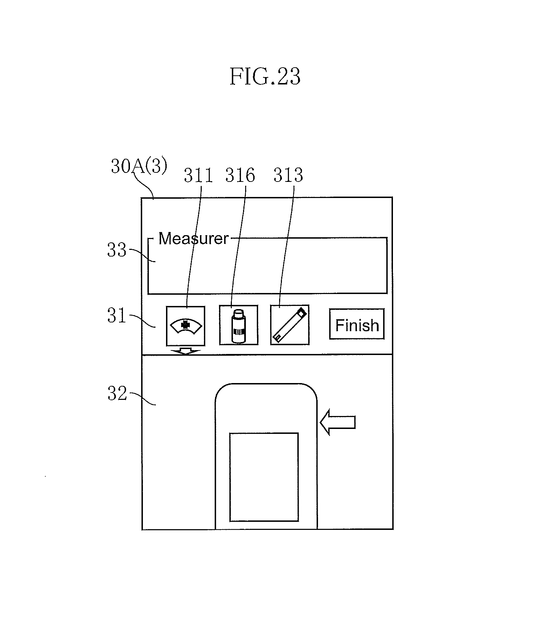

In the case where the measuring apparatus A has a function of performing quality control measurement for more accurate measurement of blood samples collected from a patient, measurement processing that employs a quality control substance ID instead of the above-mentioned patient ID may be performed. Fig. 23 shows the display unit 3 on which the individual display pattern 30A in the case of performing quality control measurement is displayed. When the measuring apparatus A is set to a mode for performing quality control measurement, a predetermined combination of attribute display portions for quality control measurement is displayed in the attribute display portion area 31. In this example, an attribute display portion 316 corresponding to the quality control substance ID is displayed in the attribute display portion area 31 in addition to the attribute display portion 311 corresponding to the measurer ID and the attribute display portion 313 corresponding to the specimen ID. The quality control substance ID identifies the quality control substance to be used in quality control, which is referred to as the control liquid, for example, and includes numerical information on blood sugar levels as measurement results to be obtained by measurement using the control liquid, for example. The user is able to grasp that the measuring apparatus A is set to the mode for performing quality control measurement and what data needs to be input, by viewing the attribute display portion area 31.

The measuring apparatus and the measuring method according to the present invention are not limited to the above-mentioned embodiments. Various design modifications can be made to the specific configurations of the measuring apparatus and the measuring method according to the present invention.

## Claims

1. A measuring apparatus comprising:
a measurement unit for performing measurement processing on a sample;
an input unit for inputting data that is associated with the measurement processing by the measurement unit and belongs to one of a plurality of attributes; and
a display unit for providing a user with information visually,
wherein the display unit displays an individual display pattern having an attribute display portion area for displaying a plurality of attribute display portions that respectively correspond to the plurality of attributes and an input instructing area for visually instructing an operation for inputting the data belonging to the attribute corresponding to one of the plurality of attribute display portions displayed in the attribute display portion area.

2. The measuring apparatus according to claim 1, wherein the attribute display portions take a plurality of visually different states, according to an input state of the data belonging to the corresponding attribute.

3. The measuring apparatus according to claim 1 or 2, wherein when input of data belonging to one attribute has been completed in a state where the attribute display portion corresponding to the attribute is displayed in the attribute display portion area, the attribute display portion corresponding to another attribute is displayed in the attribute display portion area, and an operation for inputting data belonging to the other attribute is displayed in the input instructing area.

4. The measuring apparatus according to any one of claims 1-3, wherein at least one of the data belonging to the plurality of attributes that is associated with one measurement processing is used as the data that is associated with a next measurement processing.

5. The measuring apparatus according to any one of claims 1-4, comprising:
an operation unit for accepting a user operation that causes a transition from a state where the attribute display portion corresponding to one attribute is displayed in the attribute display portion area and an operation for inputting the data belonging to the attribute is displayed in the input instructing area to a state where the attribute display portion corresponding to another attribute is displayed in the attribute display portion area and an operation for inputting the data belonging to the other attribute is displayed in the input instructing area.

6. The measuring apparatus according to any one of claims 1-5, wherein the measurement unit includes a specimen insertion portion for accepting a specimen that is provided with the measurement processing, and starts the measurement processing when the specimen is inserted into the specimen insertion portion in a state where the data belonging to a plurality of specific attributes that are sufficient for execution of the measurement processing, among the plurality of attributes, have been inputted.

7. The measuring apparatus according to any one of claims 1-5, wherein the measurement unit includes a specimen insertion portion for accepting a specimen that is provided with the measurement processing, and starts the measurement processing when the specimen is inserted into the specimen insertion portion in a state where not all of the data belonging to a plurality of specific attributes that are sufficient for execution of the measurement processing, among the plurality of attributes, have been inputted.

8. The measuring apparatus according to any one of claims 1-7, wherein the display unit has an interrupt display mode for temporally or spatially interrupting display related to the measurement processing after the measurement processing has started, and performing display for inputting data belonging to the plurality of attributes that has not been inputted.

9. The measuring apparatus according to any one of claims 1-8, wherein a combination of attributes for which the attribute display portions are displayed in the attribute display portion area, among the plurality of attributes, is settable in advance.

10. A measuring method comprising:
a measurement step of performing measurement processing on a sample;
an input step of inputting data that is associated with the measurement processing in the measurement step and belongs to one of a plurality of attributes; and
a display step of providing a user with information visually,
wherein the display step comprises displaying an individual display pattern having an attribute display portion area for displaying a plurality of attribute display portions that respectively correspond to the plurality of attributes and an input instructing area for visually instructing an operation for inputting the data belonging to the attribute corresponding to one of the plurality of attribute display portions displayed in the attribute display portion area.
